# EUROPEAN PATENT SPECIFICATION

(11) **EP 0 572 272 B1**
(45) Date of publication and mention of the grant of the patent: **11.08.1999**
(21) Application number: 93304178.2
(22) Date of filing: 28.05.1993
(51) Int. Cl.: A61L 25/00

(54) **Absorbable bone sealant**
Resorbierbare Knochendichtungsmittel
Agent de cicatrisation osseuse résorbable

(30) Priority: 29.05.1992 GB 9211432
(43) Date of publication of application: 01.12.1993
(73) Proprietor: JOHNSON & JOHNSON MEDICAL, INC., Arlington, Texas 76014 (US)
(72) Inventor: Light, Nicholas D., Doune, Perthshire (GB); Gorham, Steven D., Strathblane, Glasgow G63 9DG (GB); French, Derek A., Dunfermline, Fife KY12 7TZ (GB)
(74) Representative: Fisher, Adrian John

(56) References cited:
- EP-A- 0 251 695
- WO-A-92/00745
- WO-A-92/02238
- DE-A- 3 834 884
- FR-A- 2 410 477
- GB-A- 2 131 293

## Description

The present invention relates to an absorbable composition for use in medical treatment, in particular as a bone sealant following operative surgery.

Control of bleeding from the cut end of bone is often a necessity during surgery. Control of bleeding from sections of the skull during neurosurgery is particularly important. Control of bleeding is normally achieved by applying a haemostatic wax or paste to the cut surface of the bone. Since at least some of the wax or paste remains *in situ* following the operative procedure, the wax or paste should be biocompatible and preferably resorbable so as not to interfere with healing of the bone. It is also desirable that the wax or paste should contain humectants and other agents, such as fibrous proteins, that are known to promote wound healing.

From the late 19th century onwards control of bleeding from the cut end of bone was achieved by means of bone waxes smeared onto the cut surface of the bone. Traditionally these bone waxes comprised mainly refined beeswax and their haemostatic action was physical since the wax acted by tamponade or plugging of the cut edges of the bone. The chief drawback of such traditional bone waxes is that the wax is not absorbable by the body and remains virtually unaltered from its original state at implantation in the patient. This can result in complications of healing, such as non-healing extrusion and sinus formation, which may be associated with the presence of a non-absorbable foreign body.

A further drawback of the traditional beeswax-based bone waxes is that they can be hard, brittle and non-adherent at room temperature, especially after prolonged storage. The waxes then need to be used at elevated temperatures in order for them to have the requisite malleability, tack and adherence.

Various bone sealant compositions have been described with the objective of overcoming one or both of the above drawbacks of the beeswax-based bone sealants:

US-A-3395217 discloses bone wax compositions comprising low molecular weight ethylene copolymer waxes containing from 15 to about 40 percent by weight of a comonomer and having molecular weights in the range of from 1000 to 4000. These waxes have a consistency such that they can be kneaded between the fingers at room temperature, and a degree of tack which enables them to adhere to cut bone surfaces without being unduly difficult to manipulate. However, the bone waxes disclosed in US-A-3395217 are not absorbable.

US-A-2772999 discloses an absorbable bone wax comprising a water soluble innocuous base and free acid cellulose glycolic acid ether or free acid cellulose hydroxypropionic acid ether as a haemostatic agent. The composition also preferably contains a tackifier such as cellulose glycolic acid ether salt or cellulose hydroxypropionic acid ether salt (preferably sodium salt) and water as a plasticiser.

GB-A-1584080 and FR-A-2410477 disclose bone wax compositions that are fully absorbable and have an additional haemostatic effect over and above the physical haemostatic action of the wax. The compositions comprise from 10 to 50 percent by weight of a mixture of fibrin and collagen powders, the balance of the compositions being a water-soluble and biocompatible base. The base preferably contains dextran to function as a tackifier. The additional haemostatic effect of the compositions is ascribed to the presence of both fibrin and collagen powders. Bone waxes of this type have been adopted into clinical practice. They are sold under the Registered Trade Mark ABSELE, a typical such composition comprising 17.5% by weight of stabilised ox fibrin, 17.5% by weight of stabilised ox collagen, 8.0% by weight of Dextran 70 (i.e. dextran having a molecular weight of 60000 to 80000) and 30.0% by weight of Glycerol B.P., the balance of the composition being water. This composition has a putty-like consistency at room temperature making it easy to apply to cut bone surfaces in a spreading action using the fingers or suitable applier. The formulation of the sealant is such that it adheres well to the bone surface without peeling off onto gloves or instruments. The composition adheres well to itself, so that the amount used in an operative procedure can be added to at any time as required. The composition is absorbable, with complete absorption taking place within two to three weeks with minimal tissue reaction.

A significant drawback of the compositions disclosed in GB-A-1584080 is inadequate storage properties. The compositions must be stored under cool temperature conditions not exceeding 15°C. Exposure to temperatures in excess of 30°C irreversibly alters the structure of the compositions, resulting in a crumbly texture and reduced adherence to bone surfaces.

GB-A-2131293 disclosures pasty compositions useful as dressings in the treatment of dry sockets following tooth extraction or for other medical purposes, including use as a bone sealant. The compositions comprise collagen or another collagen-type gelatinous material, dextran and an aqueous saline solution. The particularly preferred composition contains 22% by weight of insoluble collagen, 22% by weight of solubilised collagen, 8% by weight of Dextran 70 and 48% by weight of normal saline solution. This composition is stated to be superior to the compositions disclosed in GB-A-1584080 for the purpose of dressing dry sockets because it exhibits less tendency to dissolve in oral fluids. However, this composition also suffers from the problem of poor storage properties above 15°C, resulting in deterioration in the physical properties and consistency of the paste on storage at normal ambient temperatures.

WO92/02238 describes a large range of compositions for tissue bonding and sealing in surgery. The compositions comprise: (1) a first component giving tensile strength, such as albumin, collagen or fibrin; (2) a second component to support the first component by forming a gel or a matrix, such as hyaluronic acid or a cellulose derivative; and (3) optional further components, such as dextrans or polyalcohols.

It is an object of the present invention to provide absorbable paste compositions for use as bone waxes, which compositions can be made to have optimised tack and consistency at physiological temperatures and which compositions have excellent storage properties at 30°C.

The present invention provides a composition for use in medical treatment, which composition comprises, by weight: from 10% to 70% of one or more fibrous proteins; from 1% to 20% of one or more tackifying agents selected from the group consisting of dextrans, bioabsorbable cellulose derivatives, bioabsorbable starch derivatives, polyglucosides, gelatins and polyvinyl pyrrolidone; from 0.05% to 20% of one or more mucopolysaccharides; and from 10% to 80% of an aqueous solution of a physiologically acceptable electrolyte.

The fibrous proteins may be any one or more of the known fibrous proteins such as collagen, fibrin, elastin, fibronectin, laminin or the like. The preferred weight concentration range of one or more fibrous proteins is from 30% to 50% of the composition.

The preferred fibrous protein is collagen. The collagen will typically be medical grade mature bovine collagen obtained from cow hide. Medical grade collagen is obtained from limed or unlimed hide. The raw collagen obtained by grinding or mincing the hide can be treated with enzymes such as pancreatin to remove non-collagenous proteins and fats. Other proteolytic enzymes such as trypsin and pronase may also be considered which will digest protein impurities while leaving the collagen intact. The enzyme-treated collagen is washed in saline solution to remove soluble impurities, following by rinsing in deionised water, freeze drying and milling. With suitable extensive washing procedures in salt solutions the enzyme cleaning process may be omitted. The collagen may additionally be solubilized by treatment with pepsin or by acid extraction etc. Alternative, collagen may be used in the form of ground, crushed or demineralised bone.

The tackifying agent may for example be dextran or a bioabsorbable cellulose derivative such as the sodium salt of cellulose glycolic acid ether or the sodium salt of cellulose hydroxypropionic acid ether. The tackifying agent may also be a bioabsorbable starch derivative, a polyglucoside, gelatin or collagen gel, or polyvinyl pyrrolidone. The preferred tackifying agent is Dextran 70, which is a mixture of dextrans having molecular weights in the range 60000 to 80000. The preferred weight concentration range for the tackifying agent is from 5% to 10% of the composition.

The mucopolysaccharide may be one of the known naturally occurring mucopolysaccharides such as hyaluronic acid or any salt thereof, chondroitin, chondroitin-4-sulphate, chondroitin-6-sulphate, heparin, heparan sulphate, keratan sulphate, dermatan sulphate, sulphated dextrans, or an alginate.

The mucopolysaccharide is preferably present in a weight concentration range of from 0.05% to 4% of the composition, and more preferably 0.05% to 1.0% of the composition. The preferred mucopolysaccharide is hyaluronic acid or any salt thereof. Hyaluronic acid is a naturally occurring mucopolysaccharide found in connective tissue, in the aqueous humour of the eye, and in synovial fluid. Hyaluronic acid consists essentially of alternating D-glucuronic acid and N-acetylglucosamine units. It can be isolated from animal tissues such as rooster comb, or produced by fermentation of *streptococcus* bacteria. Hyaluronic acid produced by fermentation is readily available and inexpensive,, and is therefore preferred for the practice of this invention. Hyaluronic acid having a wide range of molecular weights may be used for the present invention. Typically, the hyaluronic acid will have a molecular weight in the range from 30000 to 2 million.

The aqueous solution of a physiologically acceptable electrolyte is preferably a physiological saline, but any harmless electrolyte may be used. The physiological saline will typically be phosphate buffered to physiological pH.

The compositions according to the present invention may also contain up to 40% by weight of one or more polyhydric alcohols such as glycerol, or polyethylene glycols having molecular weights in the range 100 to 4000. The polyhydric alcohol functions as a humectant and improves the consistency and stability of the compositions. The preferred polyhydric alcohol is glycerol.

Compositions according to the present invention are haemostatic, biocompatible, non-immunogenic, absorbable, replaceable by host tissue, pliable and easy to apply. By adjustment of the amounts of the various constituents the degree of tack and malleability of the compositions may be optimised for each medical application that is envisaged.

Compositions according to the present invention may be used as or in absorbable bone sealants, in which case the amount of each constituent in the composition will be adjusted to provide a composition that is malleable at room temperature and that sticks well to cut surfaces of bone without peeling off onto gloves or instruments.

*In vivo* studies have shown that the bone sealant compositions made according to the present invention are biocompatible and fully absorbed at least as fast as the prior art bone sealants disclosed in GB-A-1584080. Furthermore, important advantage of the compositions according to the present invention is that they exhibit improved storage properties when compared with the best prior art absorbable bone sealant compositions. The compositions according to the present invention can be stored without deterioration for at least 72 weeks at 30°C. In contrast, the prior art compositions disclosed, for example, in GB-A-1584080 suffer severe deterioration after only 36 weeks' storage at 30°C. The improvement in the storage properties of the compositions according to the present invention is apparently due to the presence of the small quantities of mucopolysaccharide.

Preferred embodiments of the present invention will now be described further, by way of example only, with reference to the following examples:

### Example 1

An absorbable bone sealant composition according to the present invention is prepared as follows. First, 120mg of freeze-dried hyaluronic acid (produced by fermentation, supplied by Fermentech Ltd., approximate molecular weight 1-2 million) is dissolved in 48ml of phosphate-buffered physiological saline. Then 44g of enzyme cleaned medical grade collagen powder and 8g of Dextran 70 are dispersed in the hyaluronic acid solution by mixing. The resulting composition is cast into 2g round tablets, each of which is separately packaged in a sealed aluminium foil pouch. The pouches are sterilized by exposure to a minimum dosage of 25kGy of Cobalt 60 γ-irradiation.

### Example 2

An absorbable bone sealant composition according to the prior art disclosed in GB-A-1584080 is prepared as follows. first, 30g of glycerol B.P. and 8g of Dextran 70 are dissolved in 27ml of water. Then 17.5g of solubilised collagen (moisture content 20%) and 17.5g of fibrin powder are dispersed in the solution by mixing. The resulting composition is packaged and sterilised as described in Example 1.

### Example 3

The storage properties of the packaged and sterilised compositions prepared as in Example 1 and 2 are compared as follows. In each case the 2g pouches of the respective compositions are divided into three batches. The first batch is tested immediately to determine the baseline adhesion and extrusion properties, as described below. The second batch is stored in a cool room in which the temperature is maintained below 15°C. The adhesion and extrusion properties of samples from the second batch are measured at 12 week intervals. The third batch is stored in an incubator at 30°C. The adhesion and extrusion properties of samples from the third batch are measured at 6 week intervals.

The adhesion properties of the compositions are tested by measuring the force required to separate two serrated plates bonded together by the compositions. For this test, a 2g sample of each composition is spread between two 38mm x 26mm plates having serrated faces, which are then pressed together under a 5kg load for 30 seconds. The plates are then clamped in an INSTRON (Registered Trade Mark) Tensilometer and the force required to pull the plates apart is measured.

The extrusion properties of the compositions are tested by measuring the force required to extrude the compositions through a syringe of standard dimensions. For this test, a 1g sample of each composition is inserted into a 1ml plastic syringe (B-D Plastipak). The syringe is then clamped in an INSTRON (Registered Trade Mark) Tensilometer and the force required to expel the composition from the syringe is measured.

The results of the above measurements are shown in the accompanying figures, in which:

Figure 1 shows the extrusion and adhesion forces in Newtons as a function of the number of weeks' storage for examples stored at less then 15°C. Open circles/open squares represent adhesion data; filled circles/filled squares represent extrusion data. Squares represent data for the compositions prepared as in Example 1; circles represent data for the compositions prepared as in Example 2.

Figure 2 shows the equivalent data to that in Figure 1 for samples stored at 30°C.

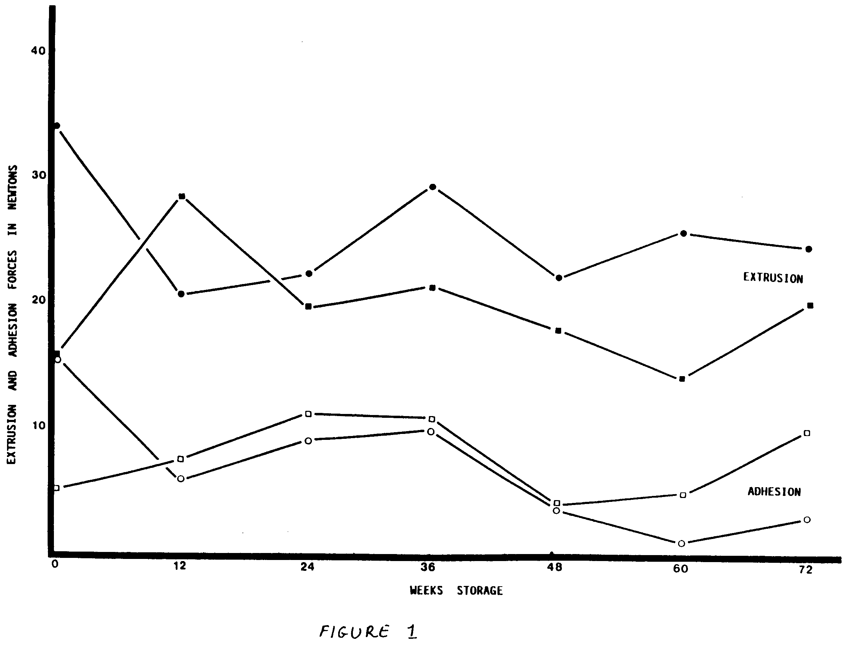

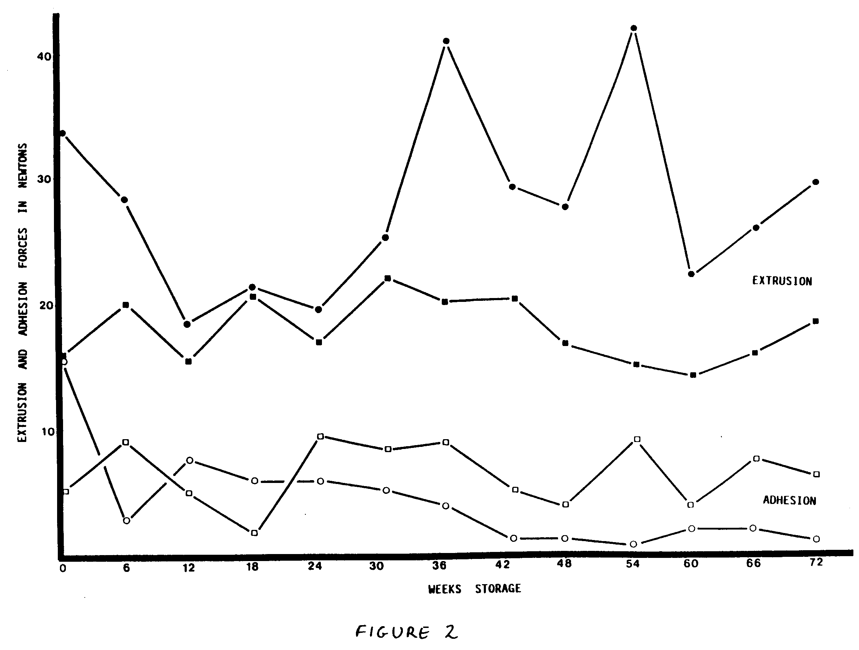

Each data point in Figures 1 and 2 is an average (mean) of at least five measurements.

The following features are apparent from the test data. First, there is an initial stabilisation period for both the compositions which results in significant changes in the adhesion and extrusion properties during the first few weeks of storage. However, following this initial stabilisation period the adhesion and extrusion properties of the composition according to the present invention made as in Example 1 remain substantially constant to within one standard deviation during 72 weeks' storage at both 15°C and 30°C. In contrast, the prior art composition made as in Example 2 shows a marked deterioration on storage at 30°C. This deterioration is manifested by the diminishing adhesion force and the high and erratic extrusion force, both of which are due to a more dry and crumbly texture of the composition after storage at 30°C. There is also in Figure 1 an indication that the adhesion force of the prior art composition decreases on prolonged storage even at temperatures below 15°C. These test data confirm the experience of users of the prior art compositions, who have found that the compositions become dry and crumbly following exposure to temperatures above 15°C. The compositions according to the present invention remain malleable and adherent even after prolonged storage at 30°C.

The above illustration of the present invention is intended by way of example only. Many other embodiments of the present invention as defined in the accompanying claims will immediately be apparent to persons skilled in the art.

## Claims

1. A composition for use in medical treatment, which composition comprises, by weight:
from 10% to 70% of one or more fibrous proteins;
from 1% to 20% of one or more tackifying agents selected from the group consisting of dextrans, bioabsorbable cellulose derivatives, bioabsorbable starch derivatives, polyglucosides, gelatin or collagen gel, and polyvinyl pyrrolidone;
from 0.05% to 20% of one or more mucopolysaccharides; and
the balance of the composition comprising from 10% to 80% of an aqueous solution of a physiologically acceptable electrolyte.

2. A composition according to claim 1 wherein the one or more fibrous proteins comprise collagen, fibrin, elastin, fibronectin or laminin.

3. A composition according to claim 1 or 2 wherein the one or more tackifying agents comprise dextran or a bioabsorbable cellulose derivative or a bioabsorbable starch derivative.

4. A composition according to any preceding claim wherein the one or more mucopolysaccharides comprise hyaluronic acid or any salt thereof, chondroitin, chondroitin-4-sulphate, chondroitin-6-sulphate, heparin, heparan sulphate, keratan sulphate, dermatan sulphate, an alginate, or sulphated dextrans.

5. A composition according to any preceding claim wherein the aqueous solution of a physiologically acceptable electrolyte is a physiological saline.

6. A composition according to any preceding claim wherein the composition further comprises up to 40% by weight of a polyhydric alcohol.

7. A composition according to claim 6 wherein the polyhydric alcohol is glycerol.

8. A composition according to any preceding claim wherein the amount of the one or more fibrous proteins is from 30% to 50% by weight.

9. A composition according to any preceding claim wherein the amount of the one or more tackifying agents is from 5% to 10% by weight.

10. A composition according to any preceding claim wherein the amount of the one or more mucopolysaccharides is from 0.05% to 4% by weight.

11. A composition according to any preceding claim wherein the amount of the one or more mucopolysaccharides is from 0.05% to 1% by weight.

12. A composition according to any preceding claim for use as an absorbable bone sealant.

13. Use of a composition according to any of claims 1 to 12 for the preparation of a medicament for reducing bleeding from cut surfaces of bones during and after surgery.

## Patentansprüche

1. Zusammensetzung zur Anwendung in einer medizinischen Behandlung, welche Zusammensetzung, in Gew.-%, umfaßt:
von 10 % bis 70 % eines oder mehrere Faserproteine,
von 1 % bis 20 % eines oder mehrerer klebrigmachender Mittel, ausgewählt aus der aus Dextranen, bioabsorbierbaren Zellulosederivaten, bioabsorbierbaren Stärkederivaten, Polyglucosiden, Gelatine- oder Kollagengel und Polyvinylpyrrolidon bestehenden Gruppe;
von 0,05 % bis 20 % eines oder mehrerer Mucopolysaccharide; und
wobei der Rest der Zusammensetzung aus 10 bis 80 % einer wäßrigen Lösung eines physiologisch annehmbaren Elektrolyten besteht.

2. Zusammensetzung nach Anspruch 1, worin das eine oder die mehreren Faserproteine Kollagen, Fibrin, Elastin, Fibronectin oder Laminin umfassen.

3. Zusammensetzung nach Anspruch 1 oder 2, worin das eine oder die mehreren klebrigmachenden Mittel Dextran oder ein bioabsorbierbares Zellulosederivat oder ein bisabsorbierbares Stärkederivat umfassen.

4. Zusammensetzung nach einem der vorstehenden Ansprüche, worin das eine oder die mehreren Mucopolysaccharide Hyaluronsäure oder ein beliebiges Salz hievon, Chondroitin, Chondroitin-4-sulfat, Chondroitin-6-sulfat, Heparin, Heparansulfat, Keratansulfat, Dermatansulfat, ein Alginat oder sulfatierte Dextrane umfassen.

5. Zusammensetzung nach einem der vorstehenden Ansprüche, worin die wäßrige Lösung eines physiologisch annehmbaren Elektrolyten eine physiologische Kochsalzlösung ist.

6. Zusammensetzung nach einem der vorstehenden Ansprüche, worin die Zusammensetzung weiterhin bis zu 40 Gew.-% eines mehrwertigen Alkohols umfaßt.

7. Zusammensetzung nach Anspruch 6, worin der mehrwertige Alkohol Glycerin ist.

8. Zusammensetzung nach einem der vorstehenden Ansprüche, worin die Menge des einen oder der mehreren Faserproteine von 30 bis 50 Gew.-% beträgt.

9. Zusammensetzung nach einem der vorstehenden Ansprüche, worin die Menge des einen oder der mehreren klebrigmachenden Mittel von 5 bis 10 Gew.-% beträgt.

10. Zusammensetzung nach einem der vorstehenden Ansprüche, worin die Menge des einen oder der mehreren Mucopolysaccharide von 0,05 bis 4 Gew.-% beträgt.

11. Zusammensetzung nach einem der vorstehenden Ansprüche, worin die Menge des einen oder der mehreren Mucopolysaccharide von 0,05 bis 1 Gew.-% beträgt.

12. Zusammensetzung nach einem der vorstehenden Ansprüche zur Anwendung als ein absorbierbares Knochenabdichtungsmittel.

13. Verwendung einer Zusammensetzung nach einem der Ansprüche 1 bis 12 zur Herstellung eines Medikaments zur Verringerung des Blutens aus Knochenschnittflächen während und nach einer chirurgischen Behandlung.

## Revendications

1. Une composition destinée à être utilisée dans un traitement médical, laquelle composition comprend, en masse :
10 % à 70 % d'une ou de plusieurs protéine(s) fibreuse(s) ;
1 % à 20 % d'un ou de plusieurs agent(s) adhésif(s) sélectionnés parmi le groupe composé de dextrans, de dérivés de cellulose biorésorbables, de dérivés d'amidon biorésorbables, de polyglucosides, d'une gélatine ou d'un gel de collagène et de pyrrolidone de polyvinyle ;
0,05 % à 20 % d'un ou de plusieurs mucopolysaccharide(s) ; et
l'équilibre de la composition comprenant 10 % à 80 % d'une solution aqueuse d'un électrolyte acceptable sur le plan physiologique.

2. Une composition selon la revendication 1, dans laquelle la ou les protéine(s) fibreuse(s) comprennent le collagène, la fibrine, l'élastine, la fibronectine ou la laminine.

3. Une composition selon la revendication 1 ou 2, dans laquelle le ou les agent(s) adhésif(s) comprennent du dextran ou un dérivé de cellulose biorésorbable ou un dérivé d'amidon biorésorbable.

4. Une composition selon l'une quelconque des revendications précédentes, dans laquelle l'un ou les mucopolysaccharide(s) comprennent l'acide hyaluronique ou l'un quelconque de ses sels, la chondroïtine, la chondroïtine-4-sulfate, la chondroïtine-6-sulfate, l'héparine, le sulfate d'héparane, le kérato-sulfate, le sulfate de dermatane, un alginate ou des dextrans sulfatés.

5. Une composition selon l'une quelconque des revendications précédentes, dans laquelle la solution aqueuse d'un électrolyte physiologiquement acceptable est un sérum physiologique.

6. Une composition selon l'une quelconque des revendications précédentes, dans laquelle la composition comprend en outre jusqu'à 40 % en masse d'un alcool polyvalent.

7. Une composition selon la revendication 6, dans laquelle l'alcool polyvalent est du glycérol.

8. Une composition selon l'une quelconque des revendications précédentes, dans laquelle la quantité de la ou des protéine(s) fibreuse(s) est comprise entre 30 % et 50 % en masse.

9. Une composition selon l'une quelconque des revendications précédentes, dans laquelle la quantité du ou des agent(s) adhésif(s) est comprise entre 5 % et 10 % en masse.

10. Une composition selon l'une quelconque des revendications précédentes, dans laquelle la quantité du ou des mucopolysaccharide(s) êst comprise entre 0,05 % et 4 % en masse.

11. Une composition selon l'une quelconque des revendications précédentes, dans laquelle la quantité du ou des mucopolysaccharide(s) est comprise entre 0,05 % et 1 % en masse.

12. Une composition selon l'une quelconque des revendications précédentes, destinée à être utilisée en tant qu'agent de cicatrisation osseuse résorbable.

13. Utilisation d'une composition selon l'une des revendications 1 à 12, pour la préparation d'un médicament destiné à réduire le saignement provenant de surfaces de sectionnement osseux au cours et à la suite d'une intervention chirurgicale.
